# EUROPEAN PATENT APPLICATION

(11) **EP 4 691 511 A1**
(43) Date of publication of application: **11.02.2026**
(21) Application number: 24806910.6
(22) Date of filing: 10.04.2024
(51) Int. Cl.: A61M 1/36

(54) **BLOOD PURIFICATION DEVICE**

(30) Priority: 18.05.2023 JP 2023082100
(71) Applicant: Nipro Corporation, Settsu-shi, Osaka 566-8510 (JP)
(72) Inventor: KAMOSHITA, Yoichi, Settsu-shi, Osaka 566-8510 (JP); WASHIO, Yukio, Settsu-shi, Osaka 566-8510 (JP); YAMAMOTO, Kazuki, Settsu-shi, Osaka 566-8510 (JP)
(74) Representative: Prüfer & Partner mbB Patentanwälte · Rechtsanwälte
(86) International application number: PCT/JP2024/014520
(87) International publication number: WO 2024/236956

(57) **Abstract**

A blood purification apparatus (100) can adjust a fluid level by storing a priming fluid (10) in a vein-side air trap chamber (117) while a vein connector (119) is connected to a connection port (161), a vein-side on/off valve (112v) is closed, and a section on a downstream side of the vein-side air trap chamber (117) in a vein-side blood circuit (112) is replaced with air in a priming process.

## Description

### TECHNICAL FIELD

The present invention relates to a blood purification apparatus.

### BACKGROUND ART

Prior-art documents that disclose a method of controlling a fluid level in a drip chamber coupled to an extracorporeal circulation circuit for blood include Japanese Patent Laying-Open No. 09-164197 (PTL 1). In the method of controlling the fluid level in the drip chamber described in PTL 1, the extracorporeal circulation circuit is substantially prevented from expanding, and a fluid is supplied to the drip chamber while an exhaust valve and an air valve coupled to the drip chamber are closed. In this state, a pressure sensor detects increase in pressure in the drip chamber to a setting pressure. As the fluid level in the drip chamber rises to a setting level, supply of the fluid is stopped and the air valve is opened. With the air valve, pressurized air stored in the drip chamber is exhausted. Upon detection of a non-pressurized state of the drip chamber, the air valve is closed and the exhaust valve is opened, and thereafter a fluid is supplied. While the drip chamber is held at a predetermined fluid level, the fluid is discharged through the exhaust valve.

### CITATION LIST

### PATENT LITERATURE

PTL 1: Japanese Patent Laying-Open No. 09-164197

### SUMMARY OF INVENTION

### TECHNICAL PROBLEM

In the method of controlling the fluid level in the drip chamber described in PTL 1, the fluid level in the drip chamber is kept track of as a function of the pressure in the drip chamber. Therefore, unless the fluid level yet to be adjusted is held constant at the setting level, the fluid level cannot accurately be adjusted.

The present invention was made in view of the problem above, and an object thereof is to provide a blood purification apparatus capable of adjusting a fluid level in a drip chamber without being affected by variation in fluid level yet to be adjusted.

### SOLUTION TO PROBLEM

A blood purification apparatus based on the present invention includes a blood purification instrument, an artery-side blood circuit, a vein-side blood circuit, a dialysis fluid supply source, a dialysis fluid pipe channel, a blood pump, a priming fluid pipe channel, a vein-side air trap chamber, a vein connector, a vein-side on/off valve, a dialysis fluid pump, a connection port, and a vein-side gas supply and exhaust pipe channel. The artery-side blood circuit is connected to the blood purification instrument and provided for inflow of blood into the blood purification instrument. The vein-side blood circuit is connected to the blood purification instrument and provided for outflow of blood from the blood purification instrument. From the dialysis fluid supply source, a fresh dialysis fluid is supplied. The dialysis fluid pipe channel is connected to the dialysis fluid supply source, and through the dialysis fluid pipe channel, the fresh dialysis fluid supplied from the dialysis fluid supply source and a used dialysis fluid used in the blood purification instrument can flow. The blood pump is provided in the artery-side blood circuit and configured to deliver blood. The priming fluid pipe channel is connected to the artery-side blood circuit, and through the priming fluid pipe channel, a priming fluid is supplied. The vein-side air trap chamber is provided in the vein-side blood circuit. The vein connector is provided in the vein-side blood circuit, and via the vein connector, a vein is accessible. The vein-side on/off valve is provided on a downstream side of the vein-side air trap chamber in the vein-side blood circuit and capable of opening and closing the vein-side blood circuit. The dialysis fluid pump is provided in the dialysis fluid pipe channel and capable of delivering the fresh dialysis fluid and the used dialysis fluid. The connection port is provided in the dialysis fluid pipe channel and connectable to the vein connector. The vein-side gas supply and exhaust pipe channel is connected to the vein-side air trap chamber, and through the vein-side gas supply and exhaust pipe channel, air in the vein-side air trap chamber can be exhausted and air can be supplied into the vein-side air trap chamber. The dialysis fluid pump is driven in a priming process to allow the fresh dialysis fluid and the used dialysis fluid to intermittently flow through the dialysis fluid pipe channel. The blood purification apparatus can adjust a fluid level by storing the priming fluid in the vein-side air trap chamber while the vein connector is connected to the connection port, the vein-side on/off valve is closed, and a section on the downstream side of the vein-side air trap chamber in the vein-side blood circuit is replaced with air in the priming process.

In one form of the present invention, the blood purification apparatus performs, in the priming process, a first priming step, in which priming is performed until a certain amount of the priming fluid is stored in the vein-side air trap chamber by forward driving the blood pump while the vein connector is connected to the connection port and the vein-side on/off valve is open, a second priming step following the first priming step, in which inside of the vein-side air trap chamber and the section on the downstream side of the vein-side air trap chamber in the vein-side blood circuit are replaced with air by stopping the blood pump and supplying air from the vein-side gas supply and exhaust pipe channel into the vein-side air trap chamber, a third priming step following the second priming step, in which the inside of the vein-side air trap chamber is maintained at an atmospheric pressure through the vein-side gas supply and exhaust pipe channel by closing the vein-side on/off valve to stop supply of air from the vein-side gas supply and exhaust pipe channel into the vein-side air trap chamber, a fourth priming step following the third priming step, in which the fluid level is adjusted by forward driving the blood pump or exhausting air in the vein-side air trap chamber through the vein-side gas supply and exhaust pipe channel to store the priming fluid in the vein-side air trap chamber, and a fifth priming step following the fourth priming step, in which a section from the vein-side on/off valve to the vein connector in the vein-side blood circuit is subjected to priming by opening the vein-side on/off valve.

In one form of the present invention, the blood purification apparatus further includes a viscous chamber where the fresh dialysis fluid and the used dialysis fluid can be stored, the viscous chamber being provided in the dialysis fluid pipe channel. The dialysis fluid pump is a viscous pump, the viscous pump being connected to the viscous chamber and capable of delivering the fresh dialysis fluid and the used dialysis fluid.

### ADVANTAGEOUS EFFECTS OF INVENTION

According to the present invention, the fluid level in the drip chamber is adjustable without being affected by variation in fluid level yet to be adjusted.

### BRIEF DESCRIPTION OF DRAWINGS

Fig. 1 is a circuit diagram showing a configuration of a blood purification apparatus according to one embodiment of the present invention.
Fig. 2 is a circuit diagram showing a state in which a first priming step is performed in the blood purification apparatus according to one embodiment of the present invention.
Fig. 3 is a circuit diagram showing a state in which a second priming step is performed in the blood purification apparatus according to one embodiment of the present invention.
Fig. 4 is a circuit diagram showing a state in which a third priming step is performed in the blood purification apparatus according to one embodiment of the present invention.
Fig. 5 is a circuit diagram showing a state in which a fourth priming step is performed in the blood purification apparatus according to one embodiment of the present invention.
Fig. 6 is a circuit diagram showing a state in which a fifth priming step is performed in the blood purification apparatus according to one embodiment of the present invention.

### DESCRIPTION OF EMBODIMENTS

A blood purification apparatus according to one embodiment of the present invention will be described below with reference to the drawings. In the description of an embodiment below, the same or corresponding elements in the drawings have the same reference characters allotted and description thereof will not be repeated.

In the description of an embodiment below, a blood purification apparatus used for continuous renal replacement therapy (CRRT) will be described as a blood purification apparatus. The blood purification apparatus may be a blood purification apparatus to be used for any of continuous hemodiafiltration (CHDF), continuous hemofiltration (CHF), continuous hemodialysis (CHD), and slow continuous ultrafiltration (SCUF).

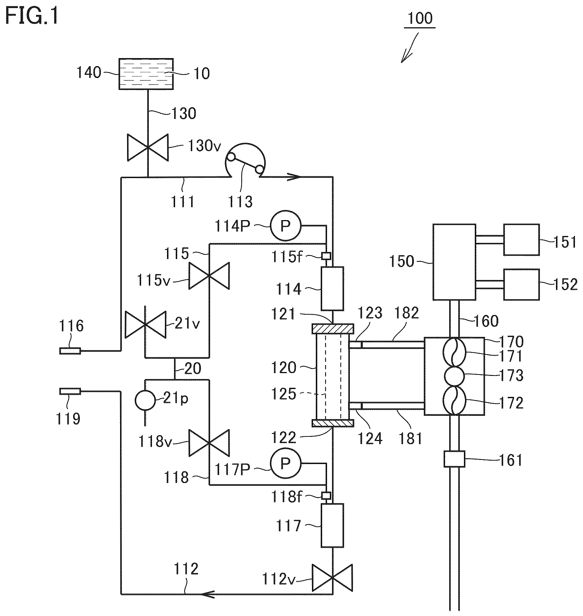

Fig. 1 is a circuit diagram showing a configuration of a blood purification apparatus according to one embodiment of the present invention. As shown in Fig. 1, a blood purification apparatus 100 according to one embodiment of the present invention includes a blood purification instrument 120, an artery-side blood circuit 111, a vein-side blood circuit 112, a dialysis fluid supply source 150, a dialysis fluid pipe channel 160, a blood pump 113, a priming fluid pipe channel 130, a vein-side air trap chamber 117, a vein connector 119, a vein-side on/off valve 112v, a dialysis fluid pump, a connection port 161, and a vein-side gas supply and exhaust pipe channel 118.

In the present embodiment, blood purification apparatus 100 further includes an artery-side air trap chamber 114, an artery connector 116, an artery-side gas supply and exhaust pipe channel 115, an artery-side air valve 115v, a vein-side air valve 118v, an artery-side pressure measurement apparatus 114P, a vein-side pressure measurement apparatus 117P, a priming fluid supply source 140, a priming on/off valve 130v, an atmospheric relief valve 21v, an air pump 21p, an artery-side protective filter 115f, a vein-side protective filter 118f, a first powder dissolving apparatus 151, and a second powder dissolving apparatus 152. At least one of these features does not necessarily have to be provided.

Blood purification instrument 120 contains a semipermeable membrane formed, for example, from a hollow fiber membrane. Blood purification instrument 120 is provided with a blood inlet 121 and a blood outlet 122. Artery-side blood circuit 111 is connected to blood inlet 121. Vein-side blood circuit 112 is connected to blood outlet 122.

Artery-side blood circuit 111 is provided with blood pump 113 that delivers blood. In artery-side blood circuit 111, artery-side air trap chamber 114 is provided between blood pump 113 and blood purification instrument 120. Artery-side air trap chamber 114 is provided with artery-side pressure measurement apparatus 114P that measures a pressure in artery-side air trap chamber 114. Artery-side protective filter 115f is provided between artery-side air trap chamber 114 and artery-side pressure measurement apparatus 114P.

Artery connector 116 is provided in artery-side blood circuit 111, and via artery connector 116, an artery is accessible. A pressure of blood taken from an artery of a patient through artery connector 116 is measured while it flows through artery-side blood circuit 111 and passes through artery-side air trap chamber 114, and thereafter blood flows into blood purification instrument 120 through blood inlet 121. Artery-side air trap chamber 114 is provided to prevent air from being introduced in blood in artery-side blood circuit 111.

Vein-side air trap chamber 117 is provided in vein-side blood circuit 112. Vein-side air trap chamber 117 is provided with vein-side pressure measurement apparatus 117P that measures a pressure in vein-side air trap chamber 117. Vein-side protective filter 118f is provided between vein-side air trap chamber 117 and vein-side pressure measurement apparatus 117P. On a downstream side of vein-side air trap chamber 117 in vein-side blood circuit 112, vein-side on-off valve 112v capable of opening and closing vein-side blood circuit 112 is provided.

Vein connector 119 is provided in vein-side blood circuit 112, and via vein connector 119, a vein is accessible. A pressure of blood purified by blood purification instrument 120 is measured while it flows through vein-side blood circuit 112 and passes through vein-side air trap chamber 117, and thereafter blood returns to a vein of the patient through vein connector 119. Vein-side air trap chamber 117 is provided to prevent air from being introduced in blood in vein-side blood circuit 112.

Blood purification instrument 120 is further provided with a dialysis fluid inlet 124 and a dialysis fluid outlet 123. A first connection pipe channel 181 is connected to dialysis fluid inlet 124. A second connection pipe channel 182 is connected to dialysis fluid outlet 123.

Priming fluid pipe channel 130 is connected to artery-side blood circuit 111, and a priming fluid 10 is supplied therethrough. Priming fluid 10 is a physiological saline or the like. An upstream end of priming fluid pipe channel 130 is connected to priming fluid supply source 140. Priming fluid supply source 140 is a bag where a physiological saline or the like is stored. Priming on/off valve 130v that opens and closes priming fluid pipe channel 130 is provided in priming fluid pipe channel 130.

Artery-side gas supply and exhaust pipe channel 115 is connected to artery-side air trap chamber 114. Artery-side air valve 115v capable of opening and closing artery-side gas supply and exhaust pipe channel 115 is provided in artery-side gas supply and exhaust pipe channel 115. Artery-side gas supply and exhaust pipe channel 115 is configured such that air in artery-side air trap chamber 114 can be exhausted therethrough and air can be supplied into artery-side air trap chamber 114 therethrough.

Vein-side gas supply and exhaust pipe channel 118 is connected to vein-side air trap chamber 117. Vein-side air valve 118v capable of opening and closing vein-side gas supply and exhaust pipe channel 118 is provided in vein-side gas supply and exhaust pipe channel 118. Vein-side gas supply and exhaust pipe channel 118 is configured such that air in vein-side air trap chamber 117 can be exhausted therethrough and air can be supplied into vein-side air trap chamber 117 therethrough.

A section on a downstream side of artery-side air valve 115v in artery-side gas supply and exhaust pipe channel 115 and a section on the downstream side of vein-side air valve 118v in vein-side gas supply and exhaust pipe channel 118 are connected to each other through a connection pipe channel 20. Atmospheric relief valve 21v is provided on the downstream side of a portion of connection to connection pipe channel 20 in artery-side gas supply and exhaust pipe channel 115. Air pump 21p is provided on the downstream side of a portion of connection to connection pipe channel 20 in vein-side gas supply and exhaust pipe channel 118. Positions where atmospheric relief valve 21v and air pump 21p are provided may be interchanged.

As artery-side air valve 115v, vein-side air valve 118v, atmospheric relief valve 21v, and air pump 21p are provided, through artery-side gas supply and exhaust pipe channel 115, air in artery-side air trap chamber 114 can be exhausted and air can be supplied into artery-side air trap chamber 114, and through vein-side gas supply and exhaust pipe channel 118, air in vein-side air trap chamber 117 can be exhausted and air can be supplied into vein-side air trap chamber 117.

Each of artery-side protective filter 115f and vein-side protective filter 118f performs a function to prevent blood from flowing out of the blood circuit and a function to prevent infection from the pressure measurement apparatus.

A fresh dialysis fluid is supplied from dialysis fluid supply source 150. The fresh dialysis fluid is prepared by mixing reverse osmosis water, a first undiluted solution, and a second undiluted solution in dialysis fluid supply source 150.

The first undiluted solution is a liquid formulation prepared by dissolving in water, first powders containing, for example, at least one of calcium salt, magnesium salt, potassium salt, and sodium salt and glucose which are at least one of solute components of the fresh dialysis fluid. The first undiluted solution is produced in first powder dissolving apparatus 151. The first undiluted solution may be stored in a first undiluted solution tank in a state produced in advance, instead of being produced in first powder dissolving apparatus 151.

The second undiluted solution is a liquid formulation prepared by dissolving in water, second powders containing at least sodium hydrogen carbonate which is another one of solute components of the fresh dialysis fluid. The second undiluted solution is produced in second powder dissolving apparatus 152. The second undiluted solution may be stored in a second undiluted solution tank in a state produced in advance, instead of being produced in second powder dissolving apparatus 152.

Dialysis fluid supply source 150 is connected to each of first powder dissolving apparatus 151 and second powder dissolving apparatus 152. Dialysis fluid supply source 150 is supplied with the first undiluted solution from first powder dissolving apparatus 151. Dialysis fluid supply source 150 is supplied with the second undiluted solution from second powder dissolving apparatus 152. **In** an example where the first undiluted solution is stored in the first undiluted solution tank, the first undiluted solution is supplied from the first undiluted solution tank to dialysis fluid supply source 150. **In** an example where the second undiluted solution is stored in the second undiluted solution tank, the second undiluted solution is supplied from the second undiluted solution tank to dialysis fluid supply source 150.

Dialysis fluid pipe channel 160 is connected to dialysis fluid supply source 150, and through dialysis fluid pipe channel 160, the fresh dialysis fluid supplied from dialysis fluid supply source 150 and a used dialysis fluid used in blood purification instrument 120 can flow. A dialysis fluid pump capable of delivering the fresh dialysis fluid and the used dialysis fluid is provided in dialysis fluid pipe channel 160.

In the present embodiment, blood purification apparatus 100 includes a viscous control system 170 including a first chamber 171, a second chamber 172, and a viscous pump 173 which is a dialysis fluid pump. Viscous control system 170 is provided in dialysis fluid pipe channel 160. Without being limited to viscous pump 173, the dialysis fluid pump should only be able to deliver the fresh dialysis fluid and the used dialysis fluid.

Each of first chamber 171 and second chamber 172 is a viscous chamber. The viscous chamber includes a fresh dialysis fluid compartment, a used dialysis fluid compartment, and a viscous compartment. As silicone oil is delivered from and into the viscous compartment with viscous pump 173, a volume of the fresh dialysis fluid compartment and the used dialysis fluid compartment can be adjusted.

First connection pipe channel 181 is a pipe channel for supply of the fresh dialysis fluid into blood purification instrument 120. Blood purification instrument 120 and viscous control system 170 are connected to each other through first connection pipe channel 181. Specifically, dialysis fluid inlet 124 of blood purification instrument 120 and the fresh dialysis fluid compartment in each of first chamber 171 and second chamber 172 of viscous control system 170 are connected to each other through first connection pipe channel 181.

Each of first chamber 171 and second chamber 172 includes a first on/off valve. While the first on/off valve of first chamber 171 is open, the first on/off valve of second chamber 172 is closed, and while the first on/off valve of first chamber 171 is closed, the first on/off valve of second chamber 172 is open. First connection pipe channel 181 is connected to the fresh dialysis fluid compartment in first chamber 171 with the first on/off valve being interposed, and connected to the fresh dialysis fluid compartment in second chamber 172 with the first on/off valve being interposed. First connection pipe channel 181 thus alternately communicates with the fresh dialysis fluid compartment in each of first chamber 171 and second chamber 172.

The used dialysis fluid used in blood purification instrument 120 flows through second connection pipe channel 182. Blood purification instrument 120 and viscous control system 170 are connected to each other through second connection pipe channel 182. Specifically, dialysis fluid outlet 123 of blood purification instrument 120 and the used dialysis fluid compartment in each of first chamber 171 and second chamber 172 of viscous control system 170 are connected to each other through second connection pipe channel 182.

Each of first chamber 171 and second chamber 172 includes a second on/off valve. While the second on/off valve of first chamber 171 is open, the second on/off valve of second chamber 172 is closed, and while the second on/off valve of first chamber 171 is closed, the second on/off valve of second chamber 172 is open. Second connection pipe channel 182 is connected to the used dialysis fluid compartment in first chamber 171 with the second on/off valve being interposed, and connected to the used dialysis fluid compartment in second chamber 172 with the second on/off valve being interposed. Second connection pipe channel 182 thus alternately communicates with the used dialysis fluid compartment in each of first chamber 171 and second chamber 172.

As set forth above, blood purification apparatus 100 according to the present embodiment includes the viscous chamber where the fresh dialysis fluid and the used dialysis fluid can be stored, the viscous chamber being provided in dialysis fluid pipe channel 160. In first chamber 171 and second chamber 172, the fresh dialysis fluid supplied from dialysis fluid supply source 150 is stored in the fresh dialysis fluid compartment. In first chamber 171 and second chamber 172, the used dialysis fluid used in blood purification instrument 120 is stored in the used dialysis fluid compartment.

The dialysis fluid pump is viscous pump 173 that is connected to the viscous chamber and capable of delivering the fresh dialysis fluid and the used dialysis fluid. As viscous pump 173 is forward driven, silicone oil moves from the viscous compartment in first chamber 171 to the viscous compartment in second chamber 172. A negative pressure is thus produced in each of the fresh dialysis fluid compartment and the used dialysis fluid compartment in first chamber 171. A positive pressure, on the other hand, is produced in each of the fresh dialysis fluid compartment and the used dialysis fluid compartment in second chamber 172.

Consequently, the fresh dialysis fluid supplied from dialysis fluid supply source 150 flows into the fresh dialysis fluid compartment in first chamber 171, and the used dialysis fluid used in blood purification instrument 120 flows into the used dialysis fluid compartment in first chamber 171 through second connection pipe channel 182.

Then, as viscous pump 173 is reverse driven, silicone oil moves from the viscous compartment in second chamber 172 to the viscous compartment in first chamber 171/ A negative pressure is thus produced in each of the fresh dialysis fluid compartment and the used dialysis fluid compartment in second chamber 172. A positive pressure, on the other hand, is produced in each of the fresh dialysis fluid compartment and the used dialysis fluid compartment in first chamber 171.

Consequently, the fresh dialysis fluid supplied from dialysis fluid supply source 150 flows into the fresh dialysis fluid compartment in second chamber 172, and the used dialysis fluid used in blood purification instrument 120 flows through second connection pipe channel 182 into the used dialysis fluid compartment in second chamber 172. Viscous pump 173 alternately repeats forward drive and reverse drive. The fresh dialysis fluid and the used dialysis fluid thus intermittently flow through dialysis fluid pipe channel 160.

Connection port 161 is provided in dialysis fluid pipe channel 160 and connectable to vein connector 119. In the present embodiment, connection port 161 is provided on the downstream side of the dialysis fluid pump in dialysis fluid pipe channel 160. In other words, connection port 161 connectable to vein connector 119 is provided on the downstream side of viscous control system 170 in dialysis fluid pipe channel 160. In an example where connection port 161 is provided on the downstream side of second connection pipe channel 182 in dialysis fluid pipe channel 160 where variation in pressure is greater, a function of the present invention is effectively achieved. Connection port 161 may be provided on an upstream side of the dialysis fluid pump in dialysis fluid pipe channel 160. As a result of connection of vein connector 119 to connection port 161, priming fluid 10 that has flowed into vein-side blood circuit 112 can be discharged to dialysis fluid pipe channel 160 through vein connector 119 and connection port 161.

Operations in performing a priming process in blood purification apparatus 100 according to the present embodiment will be described below. In blood purification apparatus 100 according to the present embodiment, the dialysis fluid pump is driven in the priming process so that the fresh dialysis fluid and the used dialysis fluid intermittently flow through dialysis fluid pipe channel 160. As the dialysis fluid pump is continuously driven in the priming process, a state of mixing between the first undiluted solution and the second undiluted solution can be maintained constant to stabilize a ratio of components in the fresh dialysis fluid supplied from dialysis fluid supply source 150.

Blood purification apparatus 100 can adjust the fluid level by storing priming fluid 10 in vein-side air trap chamber 117 while vein connector 119 is connected to connection port 161, vein-side on/off valve 112v is closed, and air in the section on the downstream side of vein-side air trap chamber 117 in vein-side blood circuit 112 is replaced with air in the priming process. Specifically, blood purification apparatus 100 performs first to fifth priming steps below in the priming process.

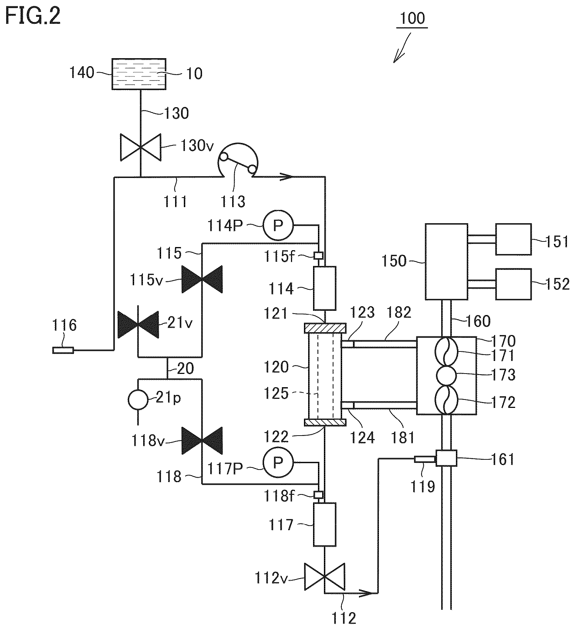

Fig. 2 is a circuit diagram showing a state in which the first priming step is performed in the blood purification apparatus according to one embodiment of the present invention. As shown in Fig. 2, in performing the priming process, vein connector 119 is connected to connection port 161.

In the first priming step, priming is performed until a certain amount of priming fluid 10 is stored in vein-side air trap chamber 117 by forward driving blood pump 113 while artery-side air valve 115v and vein-side air valve 118v are closed and vein-side on/off valve 112v is open. In the first priming step, priming on/off valve 130v is open, atmospheric relief valve 21v is closed, and air pump 21p is in a standstill.

In the first priming step, viscous pump 173 which is a dialysis pump is driven, so that the pressure at connection port 161 varies in certain cycles and consequently the fluid level of priming fluid 10 stored in vein-side air trap chamber 117 varies upward and downward.

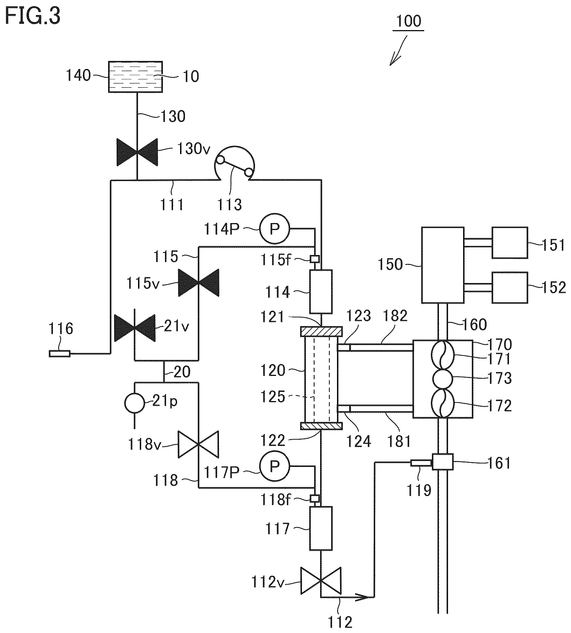

Fig. 3 is a circuit diagram showing a state in which the second priming step is performed in the blood purification apparatus according to one embodiment of the present invention. As shown in Fig. 3, in the second priming step, the inside of vein-side air trap chamber 117 and the section on the downstream side of vein-side air trap chamber 117 in vein-side blood circuit 112 are replaced with air by stopping blood pump 113 and opening vein-side air valve 118v to supply air from vein-side gas supply and exhaust pipe channel 118 into vein-side air trap chamber 117. In the second priming step, priming on/off valve 130v, artery-side air valve 115v, and atmospheric relief valve 21v are closed, and air pump 21p is forward driven to supply air into vein-side air trap chamber 117.

In the second priming step, priming fluid 10 stored in vein-side air trap chamber 117 is discharged and replaced with air. As viscous pump 173 which is the dialysis pump is driven, the pressure at connection port 161 varies in certain cycles and consequently the fluid level of priming fluid 10 varies upward and downward in the section on the downstream side of vein-side air trap chamber 117 in vein-side blood circuit 112. A section from vein-side air trap chamber 117 to vein connector 119 in vein-side blood circuit 112 does not have to completely be replaced with air, and no priming fluid 10 should only remain in vein-side air trap chamber 117 when the pressure at connection port 161 is highest.

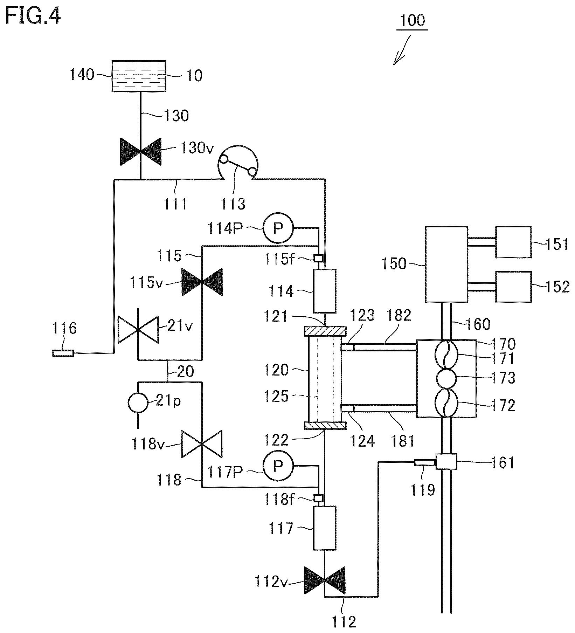

Fig. 4 is a circuit diagram showing a state in which the third priming step is performed in the blood purification apparatus according to one embodiment of the present invention. As shown in Fig. 4, in the third priming step, the inside of vein-side air trap chamber 117 is maintained at the atmospheric pressure through vein-side gas supply and exhaust pipe channel 118 by closing vein-side on/off valve 112v to stop supply of air from vein-side gas supply and exhaust pipe channel 118 into vein-side air trap chamber 117. In the third priming step, priming on/off valve 130v and artery-side air valve 115v are closed, vein-side air valve 118v and atmospheric relief valve 21v are open, and blood pump 113 and air pump 21p are in a standstill.

In the third priming step, vein-side on/off valve 112v is closed. Therefore, the pressure of air in vein-side air trap chamber 117 is maintained at the atmospheric pressure without being affected by variation in pressure at connection port 161.

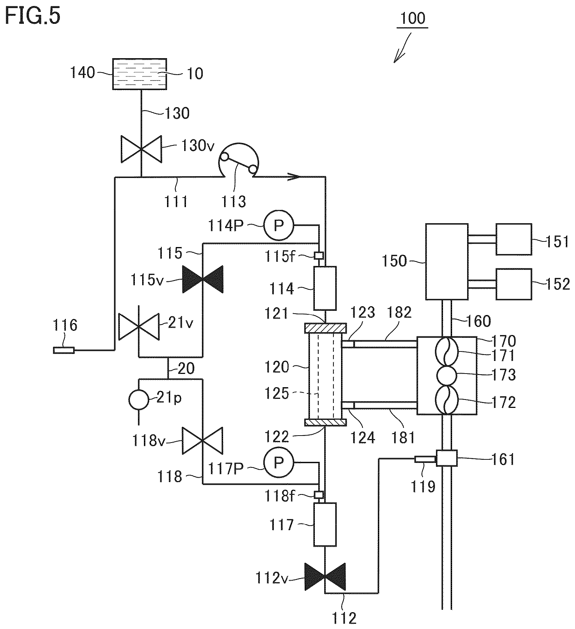

Fig. 5 is a circuit diagram showing a state in which the fourth priming step is performed in the blood purification apparatus according to one embodiment of the present invention. As shown in Fig. 5, in the fourth priming step, the fluid level is adjusted by forward driving blood pump 113 to store priming fluid 10 in vein-side air trap chamber 117. In the fourth priming step, priming on/off valve 130v, artery-side air valve 115v, vein-side air valve 118v, and atmospheric relief valve 21v are open, and air pump 21p is in a standstill.

In the fourth priming step, instead of forward driving blood pump 113, the fluid level may be adjusted by exhausting air in vein-side air trap chamber 117 through vein-side gas supply and exhaust pipe channel 118 for suction from vein-side blood circuit 112 to store priming fluid 10 in vein-side air trap chamber 117. In this case, vein-side air valve 118v is open, priming on/off valve 130v, artery-side air valve 115v, and atmospheric relief valve 21v are closed, blood pump 113 is in a standstill, and air pump 21p is reverse driven to exhaust air in vein-side air trap chamber 117.

In the fourth priming step, vein-side on/off valve 112v is closed. Therefore, the fluid level of priming fluid 10 in vein-side air trap chamber 117 can be adjusted without being affected by variation in pressure at connection port 161.

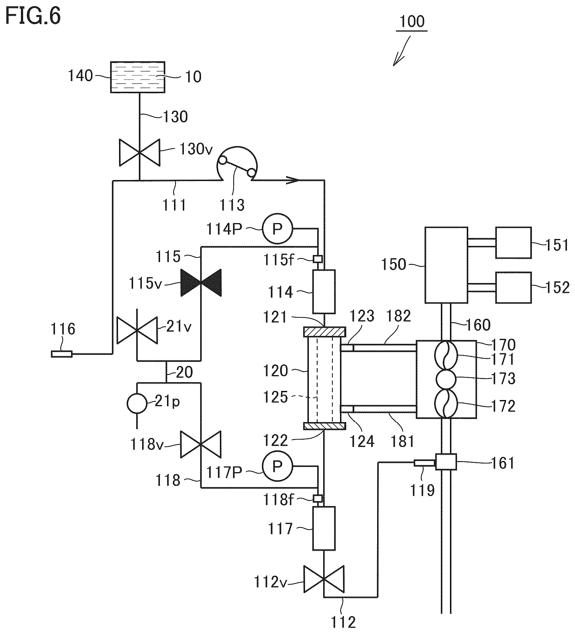

Fig. 6 is a circuit diagram showing a state in which the fifth priming step is performed in the blood purification apparatus according to one embodiment of the present invention. As shown in Fig. 6, in the fifth priming step, the section from vein-side on/off valve 112v to vein connector 119 in vein-side blood circuit 112 is subjected to priming by opening vein-side on/off valve 112v. In the fifth priming step, artery-side air valve 115v is closed, priming on/off valve 130v, vein-side air valve 118v, and atmospheric relief valve 21v are open, blood pump 113 is forward driven, and air pump 21p is in a standstill.

By performing priming as above, the fluid level in vein-side air trap chamber 117 can be adjusted without being affected by variation in fluid level in vein-side air trap chamber 117 yet to be adjusted caused by variation in pressure at connection port 161 in the first priming step.

It should be understood that the embodiment disclosed herein is illustrative and non-restrictive in every respect. The scope of the present invention is defined by the terms of the claims rather than the description above and is intended to include any modifications within the scope and meaning equivalent to the terms of the claims.

### REFERENCE SIGNS LIST

10 priming fluid; 20 connection pipe channel; 21p air pump; 21v atmospheric relief valve; 100 blood purification apparatus; 111 artery-side blood circuit; 112 vein-side blood circuit; 112v vein-side on/off valve; 113 blood pump; 114 artery-side air trap chamber; 114P artery-side pressure measurement apparatus; 115 artery-side gas supply and exhaust pipe channel; 115f artery-side protective filter; 115v artery-side air valve; 116 artery connector; 117 vein-side air trap chamber; 117P vein-side pressure measurement apparatus; 118 vein-side gas supply and exhaust pipe channel; 118f vein-side protective filter; 118v vein-side air valve; 119 vein connector; 120 blood purification instrument; 121 blood inlet; 122 blood outlet; 123 dialysis fluid outlet; 124 dialysis fluid inlet; 130 priming fluid pipe channel; 130v priming on/off valve; 140 priming fluid supply source; 150 dialysis fluid supply source; 151 first powder dissolving apparatus; 152 second powder dissolving apparatus; 160 dialysis fluid pipe channel; 161 connection port; 170 viscous control system; 171 first chamber; 172 second chamber; 173 viscous pump; 181 first connection pipe channel; 182 second connection pipe channel.

## Claims

1. A blood purification apparatus comprising:
a blood purification instrument;
an artery-side blood circuit connected to the blood purification instrument, the artery-side blood circuit being configured for inflow of blood into the blood purification instrument;
a vein-side blood circuit connected to the blood purification instrument, the vein-side blood circuit being configured for outflow of blood from the blood purification instrument;
a dialysis fluid supply source from which a fresh dialysis fluid is supplied;
a dialysis fluid pipe channel through which the fresh dialysis fluid supplied from the dialysis fluid supply source and a used dialysis fluid used in the blood purification instrument can flow, the dialysis fluid pipe channel being connected to the dialysis fluid supply source;
a blood pump provided in the artery-side blood circuit, the blood pump being configured to deliver blood;
a priming fluid pipe channel through which a priming fluid is supplied, the priming fluid pipe channel being connected to the artery-side blood circuit;
a vein-side air trap chamber provided in the vein-side blood circuit;
a vein connector via which a vein is accessible, the vein connector being provided in the vein-side blood circuit;
a vein-side on/off valve provided on a downstream side of the vein-side air trap chamber in the vein-side blood circuit, the vein-side on/off valve being capable of opening and closing the vein-side blood circuit;
a dialysis fluid pump provided in the dialysis fluid pipe channel, the dialysis fluid pump being capable of delivering the fresh dialysis fluid and the used dialysis fluid;
a connection port provided in the dialysis fluid pipe channel, the connection port being connectable to the vein connector; and
a vein-side gas supply and exhaust pipe channel through which air in the vein-side air trap chamber can be exhausted and air can be supplied into the vein-side air trap chamber, the vein-side gas supply and exhaust pipe channel being connected to the vein-side air trap chamber, wherein
the dialysis fluid pump is driven in a priming process to allow the fresh dialysis fluid and the used dialysis fluid to intermittently flow through the dialysis fluid pipe channel, and
in the priming process, a fluid level is adjustable by storing the priming fluid in the vein-side air trap chamber while the vein connector is connected to the connection port, the vein-side on/off valve is closed, and a section on the downstream side of the vein-side air trap chamber in the vein-side blood circuit is replaced with air.

2. The blood purification apparatus according to claim 1, the blood purification apparatus performing, in the priming process,
a first priming step, in which priming is performed until a certain amount of the priming fluid is stored in the vein-side air trap chamber by forward driving the blood pump while the vein connector is connected to the connection port and the vein-side on/off valve is open,
a second priming step following the first priming step, in which inside of the vein-side air trap chamber and the section on the downstream side of the vein-side air trap chamber in the vein-side blood circuit are replaced with air by stopping the blood pump and supplying air from the vein-side gas supply and exhaust pipe channel into the vein-side air trap chamber,
a third priming step following the second priming step, in which the inside of the vein-side air trap chamber is maintained at an atmospheric pressure through the vein-side gas supply and exhaust pipe channel by closing the vein-side on/off valve to stop supply of air from the vein-side gas supply and exhaust pipe channel into the vein-side air trap chamber,
a fourth priming step following the third priming step, in which the fluid level is adjusted by forward driving the blood pump or exhausting air in the vein-side air trap chamber through the vein-side gas supply and exhaust pipe channel to store the priming fluid in the vein-side air trap chamber, and
a fifth priming step following the fourth priming step, in which a section from the vein-side on/off valve to the vein connector in the vein-side blood circuit is subjected to priming by opening the vein-side on/off valve.

3. The blood purification apparatus according to claim 1 or 2, further comprising a viscous chamber where the fresh dialysis fluid and the used dialysis fluid can be stored, the viscous chamber being provided in the dialysis fluid pipe channel, wherein
the dialysis fluid pump is a viscous pump, the viscous pump being connected to the viscous chamber and capable of delivering the fresh dialysis fluid and the used dialysis fluid.
